# EUROPEAN PATENT SPECIFICATION

(11) **EP 0 781 262 B1**
(45) Date of publication and mention of the grant of the patent: **11.07.2001**
(21) Application number: 95920636.8
(22) Date of filing: 23.05.1995
(51) Int. Cl.: C07C 17/12, C07C 25/18

(54) **BROMINATION PROCESS**
BROMIERUNGSVERFAHREN
PROCEDE DE BROMINATION

(30) Priority: 16.09.1994 US 317792
(43) Date of publication of application: 02.07.1997
(73) Proprietor: ALBEMARLE CORPORATION, Baton Rouge Louisiana 70801 (US)
(72) Inventor: PARKS, John, C., Baton Rouge, LA 70817 (US); KNOEBEL, David, H., Homer, LA 71040 (US); JENKINS, Lawrence, M., Magnolia, AR 71753 (US); RANSFORD, George, H., Magnolia, AR 71753 (US); BOWMAN, Gary, L., Jr., Stamps, AR 71860 (US); HUSSAIN, Saadat, Baton Rouge, LA 70817 (US)
(74) Representative: UEXKÜLL & STOLBERG
(86) International application number: US9506565
(87) International publication number: WO9608457

(56) References cited:
- US-A- 5 030 778

## Description

This invention relates to an improved process for the bromination of diphenylalkanes.

Brominated diphenylalkanes, e.g., decabromodiphenylethane, are known flame retardants for use in polystyrene and polyolefin based thermoplastic formulations. It is predicted that decabromodiphenylethane will soon become one of the major flame retardants used by the thermoplastic industry. In response to this market opportunity, several decabromodiphenylethane processes have been proposed. See U.S. 5,077,334, U.S. 5,008,477 and U.S. 5,030,778.

US-A-5 030 778 discloses a process for preparing a decabromodiphenyl alkane, which comprises: (a) charging a reaction vessel with a bromination catalyst and liquid elemental bromine, (b) feeding liquid diphenylalkane to the reaction vessel at a point which is beneath the level of the charged liquid bromine, the liquid diphenylalkane being fed in an amount which provides from about 0.055 to about 0.033 moles of diphenylalkane per mole of elemental bromine initially charged, and (c) maintaining the reaction mass at a temperature within the range of from about 30°C to about 80°C during the liquid diphenylalkane feed. The diphenylethane can be fed as a liquid, i.e. in molten form or as a solute in a solvent. Said solvent may be among others bromine".

While these processes are quite efficacious, there is always a desire to develop more economical and technologically beneficent processes. It is an object of this invention to provide such a process.

### The Invention:

This invention provides a unique process for producing an intermediate decabromodiphenylethane slurry from which a decabromodiphenylethane wet cake can be more efficiently obtained. Even further, the obtained wet cake is most easily convertible to a high-quality ready-to-use flame-retardant product. The wet cake is characterized by having a lower occluded bromine content than that which is obtainable by prior processes.

The process of this invention comprises:
(a) in a rector providing a reaction mass containing bromine and a catalytic amount of bromination catalyst, the reaction mass being at a temperature which is in the range of 30 to 80 °C;
(b) feeding liquid diphenylethane to the reaction vessel at a point which is beneath the level of the charged reaction mass;
characterized in that
(c) the liquid diphenylethane is introduced into the reaction mass in form of a mixture formed from bromine and molten diphenylethane in a weight ratio of 5:1 to 30:1 in a flow or line mixer which is located at the end of a dip tube which extends to a point beneath the reation mass surface; and
(d) discharging a stream of the mixture from the flow or line mixer into the reaction mass, the stream having a velocity within the range of 0.3 to 30 m/s.

It is theorized, though this invention is not to be limited to a particular theory, that the feeding of the bromine/diphenylethane derived mixture, which mixture is very dilute in diphenylethane and/or underbrominated diphenylethane, to the reaction mass favorably affects the crystallization of the decabromodiphenylethane product so that there is a reduction in the formation of extremely small particles (fines) and so that there is an attenuation of the occluded free bromine content in the crystalline structure. It is believed that by having the bromine present in diluent quantities, as the derived mixture is fed, there is obtained, in the area of the feed, (1) a minimization of the variability of the brominated diphenylethane concentration, and (2) the unlikelihood that the crystallization medium, i.e., the reaction mass, will become excessively super-saturated with decabromodiphenylethane. Thus, good, slowed crystal growth is promoted and crystal nucleation is abated.

In addition, the diluent function of the fed bromine benefits product color. It is theorized that when the derived mixture is fed to the reaction mass there is a transient feed plume formed in the reaction mass. There, the diluent bromine acts as a mass transfer impediment to the bromination catalyst in the reaction mass reaching some of the yet to be brominated or underbrominated diphenylethane located in the plume. This is beneficial since it is believed that the bromination catalyst will attack, i.e., cleave, the diphenylethane -C-C- bridge if there is insufficient or no bromination of the diphenylethane prior to contact with the catalyst. The cleaved materials are, in many instances, undesirable color bodies. By impeding the mass transfer of the catalyst, for even a very short period of time, more diphenylethane will have sufficient time to obtain the degree of bromination needed to deter cleavage. Once the plume is dissipated in the reaction mass, and this occurs quickly, the bromination catalyst can then effectively catalyze the reaction to obtain the desired arbrominated decabromodiphenylethane product.

In addition to diminishing -C-C- cleavage, the dilution effect favorably affects the formation of color bodies by reducing the concentration of bromination sites per unit volume. Since the bromination sites are exothermic, the reduction in their concentration enables the avoidance of obtaining color body producing degradation temperatures at each individual sites. The large amount of bromine around each reaction site acts as heat sink so that the heat is effectively dissipated.

Generally speaking, the processes of this invention will be most useful to the commercial decabromodiphenylethane producer who deals with large reaction volumes, say larger than about 1,000 L (250 gal). Most commercial reactions will be sized from this minimum up to about 32,000 L (8,000 gal). It is in dealing with large reaction masses where the problems associated with decabromodiphenylethane production are most easily seen as mass transfer and crystallization quality are more problematic.

### Description of the Drawing:

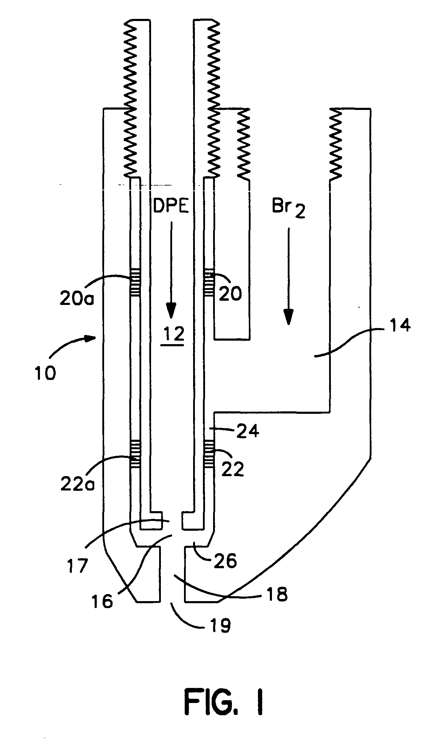

Figure 1 is a cross-sectional, partial view of a mixer suitable for use in the practice of this invention.

### Detailed Description:

The diphenylethane reactant has the formula: 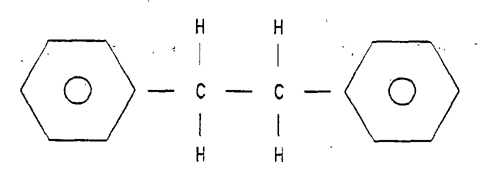 and has the IUPAC name, 1,2-diphenylethane. For convenience this compound will be referred to as simply diphenylethane.

The diphenylethane can be produced by various routes. For example, CA 97 3865d (Japanese Kokai 82/45114) and CA 46 7084g disclose the reaction of benzene and ethylene dihalide in the presence of aluminum trichloride to yield diphenylethane.

It is not uncommon for the diphenylethane to contain impurities, especially isomeric impurities, e.g., 1,1-diphenylethane. Since such impurities can adversely affect the color of the decabromodiphenylethane product, it is desirable to reduce the impurity level. Such can be done by the use of conventional purification processes, such as recrystallization wherein the diphenylethane is dissolved in a solvent and recrystallized one or more times until the purity level sought is achieved.

The diphenylethane used in forming the diphenylethane/bromine derived mixture is provided as a molten liquid due to the ease of forming an intimate mix with the bromine. To obtain the molten state, the diphenylethane is brought to a temperature which is in excess of its melting point (53° C to 55° C). Preferably, the temperature is within the range of from 55° C to 80° C (130 to 175° F). The higher temperatures are preferred as the viscosity of the molten diphenylethane will be lower, thus facilitating the formation of the derived mixture. Most preferred is a temperature within the range of from about 70° C to about 80° C (160 to 175° F).

Until it is used, it is preferred that the molten diphenylethane be blanketed by a non-oxidizing atmosphere. Such an atmosphere can be provided by most inert gases, for example, nitrogen, argon, neon, helium, krypton, xenon and the like. By providing the inert atmosphere, it has been found that the color characteristics of the final decabromodiphenylethane product are benefitted. It is theorized that this color benefit is a result of preventing or at least reducing the production of oxidation decomposition impurities in the molten diphenylethane. The decomposition impurities are probably 1-hydroxy-1,2-diphenylethane, benzaldehyde, benzyl alcohols, and the like.

The bromine/diphenylethane derived mixture can also be formed by adding solid diphenylethane to the diluent bromine.

It is preferred that the bromine used in the processes of this invention be essentially anhydrous, i.e., contain less than 100 ppm water, and contain no more than 10 ppm organic impurities, e.g., oil, grease, carbonyl containing hydrocarbons, iron, and the like. With such a bromine purity, there is little, if any, impact on the color attributes of the decabromodiphenylethane product. Available, commercial grade bromine may have such a purity. If, however, such is not available, the organic impurities and water content of the bromine can be conveniently reduced by mixing together a 3 to 1 volume ratio of bromine and concentrated (94-98 percent) sulfuric acid. A two phase mix is formed which is stirred for 10-16 hours. After stirring and settling, the sulfuric acid phase, along with the impurities and water, is separated from the bromine phase. To further enhance the purity of the bromine, the recovered bromine phase can be subjected to distillation.

The formation of the derived mixture from liquid bromine and molten diphenylethane is accomplished without substantial delay before the resultant mix is fed to the reaction mass. The technique is to form the mixture in a flow or line mixer to which the bromine and diphenylethane are fed. Exemplary of such mixers are, (1) jet mixers which depend upon the impingement of one stream into another stream, (2) injectors in which one liquid flow induces another liquid flow, (3) orifices and mixing nozzles in which the pressure drop is used to effect the mixing, (4) valves, and (5) pumps, especially centrifugal pumps into which the two streams are fed to the suction side. A common characteristic of these preferred mixers is that the mixing occurs quickly and thoroughly.

Figure 1 depicts a particularly preferred jet mixer for use in the process of this invention. The jet mixer, generally designated by the numeral 10, provides a longitudinal, axially directed conduit 12 through which the liquid diphenylethane flows. Conduit 14 carries the bromine to an annular space 24 which surrounds conduit 12. Spacers 20, 20^{a}, 22 and 22^{a} locate and hold conduit 14 in position with respect to annular space 24. At the lowermost extent of annular space 24 there is radial conduit 26 which directs the bromine flow in an inward and radial direction with respect to the long axis of conduit 12. Adjacent diphenylethane discharge port 17 and radial conduit 26 is impingement chamber 16. Downstream from impingement chamber 16 is mixing chamber 18 and mixture discharge port 19.

In operation, bromine flows through conduit 14, annular space 24 and radial conduit 26 to reach impingement chamber 16. At impingement chamber 16 the bromine is traveling in an inward and radial direction. The diphenylethane flows down conduit 12 and through discharge port 17 in an axial direction with respect to impingement chamber 16. The thus flowing diphenylethane intersects and impinges perpendicularly with the flowing bromine from radial conduit 26. Subsequent to the impingement, the resulting mix flows into mixing chamber 18 and is then discharged with velocity as a stream from the mixer.

The mixer dimensions determine the velocity of the stream from the mixer and the residency time of the bromine/ diphenylethane derived mix in the mixer. For any desired velocity and residency time, these dimensions can be conventionally determined. For example, on a commercial scale, for a 272 kg/hr (600 lb/hr) molten diphenylethane and a 2,400 kg/hr (5,300 lb/hr) bromine feed rate and for a mixer residency time of about 0.01 second. The height of radial conduit 26 can be 0.635 cm (1/4 inch) while mixing chamber 18 can be 0.80 cm (5/16 inch) in diameter and 1.9 cm (3/4 inch) long.

The weight ratio of bromine to diphenylethane which provides the desired diphenylethane and/or underbrominated diphenylethane dilution lies within the range of from 5:1 to 30:1, and is more preferably within the range of from 9:1 to 15:1. Weight ratios in excess of 30:1 may be used, however such excess ratios will result in more liquid bromine being present after the reaction.

The derived mixture will be comprised of bromine, and one or more of diphenylethane, mono-, di-, tri- and tetrabromodiphenylethane. The exact composition of the derived mixture before it is fed to the reaction mass will depend upon the time that has lapsed between the formation of the mixture and its feed to the reaction mass. The more brominated species will be present in larger quantities as the time period increases since there will be sufficient time for the additional bromination to occur. In those cases where the time period is short, the derived mixture will contain more diphenylethane and/or the lower brominated species.

It is to be understood that it is permissible to have present in the derived mixture components other than bromine, diphenylethane and brominated diphenylethane provided that these components do not defeat the very reason for practicing the processes of this invention. For example, it is best that the derived mixture not contain a bromination catalyst as such catalyst can cause the above discussed -C-C- cleavage in the unprotected species. However, if the practitioner wishes to use a bromine recycle stream from a previous process run, then it is possible that the recycled bromine could contain some small concentration of a still active bromination catalyst. Considering the small amount of catalyst present, the use of such a bromine would be permissible as the deleterious impact of the catalyst, if any, would be so small as to be acceptable in view of the effect sought.

The bromine temperature should be sufficiently high so that the molten diphenylethane is not cooled to be a solid. For most all cases, the bromine temperature is preferably within the range of from 30° C to 75° C.

In commercial systems process efficiency is paramount. Thus, the feed rate of the derived mixture should be as high as is possible in view of the capacity of the reaction system to handle evolved HBr. The feed stream cross sectional area/velocity are selected so as to expend as much energy as possible in the mixing process. It has been found that smaller cross sectional areas and faster feed stream velocities will generally yield larger crystals, lower occluded bromine in the product and a better colored product. There are practical limits, however, and they are set by cost and benefit balances and available equipment, e.g. feed pumps and overhead capacity. Exemplifying suitable cross sectional areas and velocities are: the areas within the range of from 0.03 to 10 cm² (0.0005 to 1.6 inches²) and the velocities within the range of from 0.3 to 30 m/sec (0.98 to 98 ft/sec). Such ranges are suitable for most stirred commercial reactors, and are especially suitable for 1000 to about 30,000 L (260 to about 8000 gal) reaction masses, all having the appropriate overhead capacities to handle and separate evolved Br₂ and HBr from their respective reaction masses.

The derived mixture can be fed by using one or more feed streams. In those cases where there are a plurality of streams, each stream is proportioned to handle its fraction of the feed service.

The derived mixture feed stream is introduced into the reactor below the surface of the reaction mass, say from 2.5 cm (1 inches) to 60 cm (24 inches) for a commercial scale reactor.

The reactor to which the derived mixture is fed will generally be an agitated, glass-lined reactor to which has already been charged bromine and bromination catalyst.

The charged bromination catalyst is preferably AlCl₃ and/or AlBr₃, although use may be made of aluminum powder, iron powder, FeCl₃ and FeBr₃, alone or in combination with the aluminum trihalides. Other bromination catalysts are suitable provided that they have sufficient catalytic activity to provide for the arperbromination needed to yield a decabromodiphenylethane product. Catalytic quantities are used. Typically, the catalysts will be present in an amount within the range of from 0.1 to 20 weight percent, based on the weight of the diphenylethane fed and the catalytic activity. A preferred amount is within the range of from 2 to 15 weight percent on the same basis. When AlCl₃ is the catalyst, for example, from 5.0 to 7.0 weight percent will be most preferred.

The bromination catalyst and bromine can be charged to the reaction vessel in any order or together. After charging, the bromine/catalyst mix will preferably be brought to a temperature within the range of 50° C to 60° C (122 to 140° F). Lower or higher temperatures are possible, but, lower temperatures may result in lower bromination while higher temperatures will require pressurized operation.

The amount of bromine in the reaction mass during the feed of the derived mixture is that amount which is sufficient to yield a stirrable reaction mass and, ultimately, a decabromodiphenylethane product. Such a product is defined as a mixture of brominated diphenylethanes having an average bromine number of at least about 9.0 and preferably within the range of from 9.5 and 10. There are two sources of bromine which will contribute bromine to the reaction mass, the bromine which accompanies the derived mixture and the bromine which is initially present in the reactor before the feed. The amount of bromine initially present in the reactor is preferably within the range of from 25 to 150% of the stoichiometric amount based upon the planned diphenylethane feed. Most preferred is a bromine amount within the range of from 75% to 100% of the stoichiometric amount.

During the feed of the bromine/diphenylethane derived mixture, the reaction mass temperature is kept within the range of from 30° C to 80° C, and preferably within the range of from 50° C to 60° C. Since the bromination of diphenylethane is exothermic, cooling of the reaction mass is needed to maintain the reaction mass temperature chosen. The heat of reaction can be removed from the reaction mass by cooling the reaction vessel or by having the reaction mass under reflux conditions so that heat can be removed by the use of an overhead condenser.

It is preferred that the pressure in the reaction vessel be that which provides a refluxing condition at the selected reaction mass temperature. With a refluxing condition, control of the reaction mass temperature is facilitated. If temperature control is effected otherwise, i.e., by the use of heating or cooling jackets, then the pressure can be any which is not prohibitive of the obtainment of the various defined parameters of the process. Also, since temperatures above the boiling point of bromine are useful in the process of this invention, super atmospheric pressures, e.g., 204.8 KPa (15 psig) can be used to obtain same.

The process of this invention is benefitted by the fact that, after the derived mixture feed to the reactor is completed, no further lengthy maintenance of the formed reaction mass will be needed to complete the bromination reaction to obtain the decabromodiphenylethane product. The continuance of the bromination reaction after the feed has been completed is best monitored by detecting HBr evolution from the reaction mass. Cessation of HBr evolution signals the end of the bromination reaction. With the process of this invention the bromination reaction is usually finished within one minute of the cessation of the derived mixture feed. Thus, product recovery can occur quite soon after the derived mixture feed is completed. It is to be understood that the practitioner can wait to recover the product as no harm is done except that process cycle time is extended.

After the bromination reaction has at least substantially ceased, the reaction mass will comprise a liquid-solid mixture. The solid comprises a precipitate which includes decabromodiphenylethane product, occluded free bromine and other impurities. The liquid will comprise mostly bromine and catalyst. Before recovering the solid, it is preferred to first deactivate the catalyst. Deactivation can be accomplished by introducing water to the reaction mass or vice versa. Generally, it is preferred to move the reaction mass from the reactor to a stripping vessel. The stripping vessel will contain water which (1) deactivates the catalyst and (2) which will provide for the formation of a slurry after the bromine is stripped off.

Once in the stripping vessel, the reaction mass, which comprises the solids and the bromine, forms one phase and the water, the other phase. The water phase is on top. The vessels contents are heated to boil-off or strip the bromine. The stripping temperature is generally around 57° C (135° F). The bromine boils off and passes through the water phase and takes a small amount of water with it. As more bromine is stripped, the solids and bromine phase begins to thicken. At the point where there is approximately an equal weight of bromine and solids, the solids will begin to transit into the water phase. As more bromine is stripped, the remaining solids will slurry with the water. The solids, principally decabromodiphenylethane product, do not wet well in water. The larger decabromodiphenylethane product particles tend to settle in the water. The fines, and there are always some, do not, for the most part, settle, but instead move to the top of the water surface and form a froth. The more fines, the worse the froth formation and the slower the stripping must proceed. Since the practice of the process of this invention results in an abatement of the fines production, there is an attendant reduction in froth production. Thus, it is possible, with the practice of this invention, to use a higher stripping rate. Increases in stripping rates of up to 100% have been experienced by the practice of this invention.

After the bromine has been stripped off, the water and solids slurry is treated with an aqueous base to neutralize any HBr present. The aqueous base can be any suitable base, e.g., an aqueous solution of NaOH or Na₂CO₃.

After the neutralization step, the slurry in the stripper vessel is removed therefrom and is processed to separate the liquid portion from the solids portion. This is most easily accomplished by centrifuging. It is at this centrifuging step that another process benefit is realized due to the reduction of fines production. When a large quantity of fines are present, the centrifuging time is substantially lengthened as the fines interfere with the separating movement of water from the solids portion of the slurry. By having a low fines production, there has been seen a 50-60% reduction in centrifugation times.

The solids obtained from the liquid portion are unique in that they have a relatively low occluded free bromine content, say from 500 ppm to 2000 ppm and most probably from 900 ppm to 1200 ppm. Compare this against processes which do not use highly diluted diphenylethane feed and which have a high, e.g., 3500 ppm, occluded free bromine content.

The undried solids recovered from the slurry are referred to herein by the term "wet cake". This term is not meant to be restricted by any particular manner of solids recovery and/or by ancillary treatments of the slurry or recovered solids, e.g., neutralization, washing and the like. Most often the wet cake is recovered from the filter media as a cake-like material.

The term "occluded free bromine" refers to that bromine which is tightly held by the recovered decabromodiphenylethane product component of the wet cake so that ordinary washing techniques are insufficient to reduce its content in the product. Reduction of the occluded free bromine content requires more vigorous treatment, such as by drying and grinding the wet cake in a Raymond Mill. Further reduction can be effected by the use of heat, i.e., oven-aging, the dried and ground wet cake at elevated temperatures over time. Note that the temperatures used in the stripping technique described above are not useful in effecting the reduction of the occluded free bromine content.

After obtainment of the wet cake, it is preferable to subject it to drying and grinding so as to obtain a dry particulate product of preferred size and enhanced color. There can also be realized a small, but useful further reduction in the occluded free bromine content. Reductions of about 150 ppm are obtainable. With regard to color, compare a Yellowness (YI), as measured by ASTM D-1925, of about 24 for the dried and ground wet cakes produced by processes not of this invention against a YI of about 14.5 for dried and ground wet cakes of this invention.

To further improve the color and reduce the occluded free bromine content of the ground and dried product, the product is oven-aged at a temperature within the range of from 175° C to 290° C (350° F to about 550° F) for 2 to 10 hours. A comparison against decabromodiphenylethane processes which use diphenylethane only feeds shows that such processes need longer aging times, 6 to 20 hours, to obtain the same level of occluded free bromine in the final product.

The oven-aging produces the final decabromodiphenylethane product which will be principally comprised (from 95 wt% to 99.5 wt%) of decabromodiphenylethane and smaller amounts of nonabromodiphenylethane, octabromodiphenylethane, and decabromostilbene with an occluded free bromine content within the range of from 100 ppm to 300 ppm.

The decabromodiphenylethane product of this invention may be used as a flame retardant in formulation with virtually any flammable material. The material may be macromolecular, for example, a cellulosic material or a polymer. Illustrative polymers are: olefin polymers, cross-linked and otherwise, for example, homopolymers of ethylene, propylene, and butylene; copolymers of two or more of such alkylene monomers and copolymers of one or more of such alkylene monomers and any other copolymerizable monomers, for example, ethylene/propylene copolymers, ethylene/ethyl acrylate copolymers and ethylene/vinyl acetate copolymers; polymers of olefinically unsaturated monomers, for example, polystyrene, e.g., high impact polystyrene, and styrene copolymers; polyurethanes; polyamides; polyimides; polycarbonates; polyethers; acrylic resins; polyesters, especially poly(ethyleneterephthalate) and poly(butyleneterephthalate); epoxy resins; alkyds; phenolics; elastomers, for example, butadiene/styrene copolymers and butadiene/acrylonitrile copolymers; terpolymers of acrylonitrile, butadiene and styrene; natural rubber; butyl rubber; and polysiloxanes. The polymer may also be a blend of various polymers. Further, the polymer may be, where appropriate, cross-linked by chemical means or by irradiation.

The amount of decabromodiphenylethane product used in a formulation will be that quantity needed to obtain the flame retardancy sought. It will be apparent to the practitioner that for all cases no single precise value for the proportion of the product in the formulation can be given since this proportion will vary with the particular flammable material, the presence of other additives and the degree of flame retardancy sought in any given application. Further, the proportion necessary to achieve a given flame retardancy in a particular formulation will depend upon the shape of the article into which the formulation is to be made, for example, electrical insulation, tubing and film will each behave differently. In general, however, the formulation may contain from 5 to 40 weight percent, preferably 10 to 30 weight percent, of the product when it is the only flame retardant compound in the formulation.

It is especially advantageous to use the flame retardant final product of this invention with an inorganic compound, especially ferric oxide, zinc oxide, zinc borate, the oxide of a Group V element, for example, bismuth, arsenic, phosphorus and especially antimony, in the formulation. Of these compounds, antimony oxide is especially preferred. If such a compound is present in the formulation, the quantity of product needed to achieve a given flame-retardancy is accordingly reduced. Generally, the product and the inorganic compound are in a weight ratio of from 1:1 to 7:1, and preferably of from 2:1 to 4:1.

Formulations containing a flame retardant system comprised of the product of this invention and the above inorganic compounds may contain up to about 40 percent by weight of the system and preferably between 20 percent and 30 percent by weight.

Any of the additives usually present in formulations, e.g., plasticizers, antioxidants, fillers, pigments, and UV stabilizers can be used in formulation with the product of this invention.

Thermoplastic articles formed from formulations containing a thermoplastic polymer and a product of this invention can be produced conventionally, e.g., by injection molding, extrusion molding, compression molding.

The following Examples merely illustrate the invention described herein and are not to be taken as limiting such inventions.

### EXAMPLE I

To a 15,140 L (4,000 gal) glass lined agitated reactor is added 12,712 kg (28,000 lb) of bromine. The agitator was then started and 68 kg (150 lb) AlCl₃ was added. The reaction content was heated to 58° C (135° F).

Bromine flow through a mixer was established at 2,406 kg/hr (5300 lb/hr). A bromine heater was used to obtain a temperature for the bromine of between 50° C and 55° C (120° F and 130° F). Molten diphenylethane flow to the mixer was then established at 272 kg/hr (600 lb/hr).

The mixer was located at the end of a dip tube which extended to a point 60 cm (24 inches) beneath the reaction mass surface. The mixer was of the type shown in Figure 1 hereof. The mixer was designed to handle 272 kg/hr (600 lb/hr) diphenylethane and 2406 kg/hr (5300 lb/hr) bromine. The mixer forced the bromine radially inward into a 0.48 cm (3/16 inch) stream of diphenylethane. The mixer provides a mixing chamber through which the resultant intimate mix then passes. The chamber had a 0.8 cm (5/16 inch) diameter and was 1.9 cm (3/4 inch) long. The stream leaving the mixer had a diameter of about 0.8 cm (5/16 inch) and a velocity of about 6.1 m/sec (20 ft/sec).

The reaction mass temperature rose to reflux temperature at a reaction pressure of 2.05 x 105 PA (15 psig).

The diphenylethane and bromine were allowed to flow until 1090 kg (2400 lb) of diphenylethane had been fed. Subsequently, the diphenylethane flow was stopped and nitrogen was blown through the diphenylethane passages. The bromine flow was stopped and nitrogen was blown through its passages.

The reaction mass was transferred to a stripping vessel to which had been previously added 6000 L (1500 gal) of water. The resulting mix was then heated and stirred to a temperature of about 58° C (135° F) so as to obtain the boil-off of the bromine. A 1% aqueous solution of Aerosol™-OTB made by American Cyanamide was used as a wetting agent. The boil-off period lasted about 4 hours.

After the boil-off, the remaining solids and water slurry was treated with a base to neutralize and acidic components. The neutralized slurry was fed over a 2.5 hour period to a centrifuge to yield a wet cake. The wet cake was then recovered and fed to a Raymond Mill wherein the wet cake was dried and ground. The wet cake was dried at a temperature of 205° C (400° F) for a period of 2 seconds and ground to an average particle size of from about 80 to about 4.5 microns.

The foregoing procedure was repeated several times except that in three of the runs, 23-26 minutes was used to get the bromine flow and temperature established. In another run, 4 minutes was used.

Comparative runs were made in which the diphenylethane was fed to the reactor alone.

### Comparative Runs

To a 15,140 L (4,000 gal) glass lined agitated reactor was added 21,760 kg (48,000 lb) of bromine. The agitator was then started and 68 kg (150 lb) AlCl₃ was added. The reactor contents were heated to 58° C (135° F).

Molten diphenylethane flow to a dip tube was then established at 272 kg/hr (600 lb/hr). The dip tube extended to a point 120 cm (48 inches) beneath the reaction mass surface.

The diphenylethane was allowed to flow until 1089 kg (2400 lb) diphenylethane had been fed. Subsequently, the diphenylethane flow was stopped and nitrogen was blown through the diphenylethane dip tube.

The reaction mass was transferred to a stripping vessel to which had been previously added 6000 L (1500 gal) of water. The resulting mix was then heated and stirred to a temperature of about 58° C (135° F) so as to obtain the boil-off of the bromine. A 1% aqueous solution of Aerosol™-OTB made by American Cyanamide was used as a wetting agent. The boil-off period lasted about 8 hours.

After the boil-off, the remaining solids and water slurry was treated with a base to neutralize and acidic components. The neutralized slurry was fed over a 5 hour period to a centrifuge to yield a wet cake. The wet cake was then recovered and fed to a Raymond Mill wherein the wet cake was dried and ground. The wet cake was dried at a temperature of 205° C (400° F) for a period of 2 seconds and ground to an average particle size of from about 80 to about 4.5 microns.

Measurements were obtained from all of the runs for (1) the bromine boil-off rate, (2) the centrifugation rate, (3) the occluded free-bromine content of the wet cake before and after Raymond Milling, and (4) the color values of the Raymond Milled product.

Using the process of this invention resulted in an average 75% increase in the boil-off rate and an average 97% increase in the centrifugation rate. The wet cake from the comparative runs had, on average, 78% (8 samples, 2,007 to 5,463 ppm, avg. 3,773 ppm occluded Br₂) more occluded free bromine than did the wet cakes produced by the process of this invention (8 samples, 1,308 to 838 ppm, avg. 1,122 ppm occluded Br₂). After Raymond Milling, the occluded free bromine content of the comparatively dried and ground products was, on average, 225% higher than for the dried and ground products of this invention. Compare, for the comparative 8 samples, 1,331 to 4,771 ppm, avg. 2,919 ppm occluded Br₂. Based upon the Yellowness Index (YI), the Raymond Milled products of this invention have a better color than the comparative products. The YI values, ASTM D-1925, for the dried and milled material produced in accordance with this invention, prior to oven-aging, lie within the range of from 12.5 to 17.5.

## Claims

1. A process for the manufacture of a decabromodiphenylethane product, which process comprises:
(a) in a rector providing a reaction mass containing bromine and a catalytic amount of bromination catalyst, the reaction mass being at a temperature which is in the range of 30 to 80 °C;
(b) feeding liquid diphenylethane to the reaction vessel at a point which is beneath the level of the charged reaction mass;
characterized in that
(c) the liquid diphenylethane is introduced into the reaction mass in form of a mixture formed from bromine and molten diphenylethane in a weight ratio of 5:1 to 30:1 in a flow or line mixer which is located at the end of a dip tube which extends to a point beneath the reation mass surface; and
(d) discharging a stream of the mixture from the flow or line mixer into the reaction mass, the stream having a velocity within the range of 0.3 to 30 m/s.

2. The process of claim 1 wherein the weight ratio is within the range of from 9:1 to 15:1.

3. The process of claims 1 or 2 wherein the bromination catalyst is AlCl₃, AlBr₃ or a mixture thereof.

4. The process of any of claims 1 to 3 wherein the initial amount of bromine in the reaction mass before the mixture feed begins is within the range of from 25% to 150% of the stoichiometric amount needed to produce a decabromodiphenylethane product from the diphenylethane to be fed.

5. The process of claim 4 wherein the initial amount of bromine in the reaction mass before the mixture feed begins is within the range of 75 to 100% of the stoichiometric amount needed to produce a decabromodiphenylethane product from the diphenylethane to be fed.

## Patentansprüche

1. Verfahren zur Herstellung eines Decabromodiphenylethanprodukts, bei dem
(a) in einem Reaktor eine Reaktionsmasse bereitgestellt wird, die Brom und eine katalytische Menge Bromierungskatalysator enthält, wobei sich die Reaktionsmasse auf einer Temperatur im Bereich von 30 bis 80°C befindet,
(b) flüssiges Diphenylethan an einem Punkt, der sich unter der Füllhöhe der eingebrachten Reaktionsmasse befindet, in das Reaktionsgefäß eingespeist wird,
dadurch gekennzeichnet, dass
(c) das flüssige Diphenylethan in Form einer Mischung in die Reaktionsmasse eingebracht wird, die aus Brom und geschmolzenem Diphenylethan in einem Gewichtsverhältnis von 5:1 bis 30:1 in einem Durchfluss- oder Leitungsmischer, der am Ende des Tauchrohrs angeordnet ist, das sich bis zu einem Punkt unter der Oberfläche der Reaktionsmasse erstreckt, gebildet worden ist, und
(d) ein Strom der Mischung aus dem Durchfluss- oder Leitungsmischer in die Reaktionsmasse abgegeben wird, wobei der Strom eine Geschwindigkeit im Bereich von 0,3 bis 30 m/s hat.

2. Verfahren nach Anspruch 1, bei dem das Gewichtsverhältnis im Bereich von 9:1 bis 15:1 liegt.

3. Verfahren nach Anspruch 1 oder 2, bei dem der Bromierungskatalysator AlCl₃, AlBr₃ oder eine Mischung derselben ist.

4. Verfahren nach einem der Ansprüche 1 bis 3, bei dem die Anfangsmenge Brom in der Reaktionsmasse, bevor die Mischungszufuhr beginnt, im Bereich von 25 % bis 150 % der stöchiometrischen Menge liegt, die zur Herstellung eines Decabromodiphenylethanprodukts aus dem einzuspeisenden Diphenylethan erforderlich ist.

5. Verfahren nach Anspruch 4, bei dem die Anfangsmenge Brom in der Reaktionsmasse, bevor die Mischungszufuhr beginnt, im Bereich von 75 % bis 100 % der stöchiometrischen Menge liegt, die zur Herstellung eines Decabromodiphenylethanprodukts aus dem einzuspeisenden Diphenylethan erforderlich ist.

## Revendications

1. Procédé de préparation de décabromodiphényléthane, ledit procédé comprenant les étapes consistant à :
(a) placer dans un réacteur une masse réactionnelle contenant du brome et une quantité catalytique de catalyseur de bromation, la masse réactionnelle étant à une température comprise entre 30 et 80°C;
(b) introduire du diphényléthane liquide dans le réacteur en un point qui se situe en dessous du niveau de la masse réactionnelle chargée;
cette étape (b) étant caractérisée en ce que
(c) le diphényléthane liquide est introduit dans la masse réactionnelle sous forme d'un mélange formé de brome et de diphényléthane fondu en un rapport en poids de 5:1 à 30:1 par un mélangeur en continu à courant, ou en ligne, qui est placé à l'extrémité d'un tube plongeur qui s'étend jusqu'à un point situé sous la surface de la masse réactionnelle ; et
(d) évacuer un flux du mélange par le mélangeur en continu à courant, ou en ligne, vers la masse réactionnelle, l'écoulement ayant une vitesse comprise entre 0,3 et 30 m/s.

2. Procédé selon la revendication 1 dans lequel le rapport en poids est compris entre 9:1 et 15:1.

3. Procédé selon la revendication 1 ou 2 dans lequel le catalyseur de bromation est AlCl₃, AlBr₃ ou un mélange de ceux-ci.

4. Procédé selon l'une quelconque des revendications 1 à 3 dans lequel la quantité initiale de brome dans la masse réactionnelle avant le début de l'introduction du mélange est comprise entre 25 % et 150 % de la quantité stoechiométrique nécessaire pour obtenir un produit de décabromodiphényléthane à partir du diphényléthane à introduire.

5. Procédé selon la revendication 4 dans lequel la quantité initiale de brome dans la masse réactionnelle avant le début de l'introduction du mélange est comprise dans l'intervalle entre 75 et 100 % de la quantité stoechiométrique nécessaire pour obtenir un produit de décabromodiphényléthane à partir du diphényléthane à introduire.
